# EUROPEAN PATENT APPLICATION

(11) **EP 1 800 673 A2**
(43) Date of publication of application: **27.06.2007**
(21) Application number: 06026477.7
(22) Date of filing: 20.12.2006
(51) Int. Cl.: A61K 31/045, A61K 31/105, A61K 31/305, C07K 16/00, A61P 7/06, A61P 37/00, A61P 19/02, A61P 21/04

(54) **Nitrophenyls and related compounds and thimerosal for the inhibition of immune related cell or tissue destruction**

(30) Priority: 23.12.2005 US 752912 P
(71) Applicant: Canadian Blood Services, Ottawa, Ontario K1G 4J5 (CA)
(72) Inventor: Branch, Donald R., c/o Canadian Blood Services, Ontario M5G 2M1 (CA); Lazarus, Alan H., c/o St. Michael's Hospital, Toronto, Ontario M5B 1W8 (CA); Crow, Andrew, c/o Canadian Blood Services, Ottawa, Ontario K1G 4J5 (CA)
(74) Representative: Kuhnen & Wacker

(57) **Abstract**

Composition containing a nitrophenyl compound or thimerosal for inhibiting phagocytosis of blood cells. The use of a nitrophenyl compound or thimerosal to inhibit phagocytosis of blood cells in a host having an auto or alloimmune disease.

## Description

### FIELD OF THE INVENTION

The present invention relates to methodology and compounds useful to augment the efficacy and in instances replace IVIg and anti-D therapies for immune cytopenias. More specifically, the present invention relates to the use of nitrophenyl compounds and thimerosal and congeners thereof in methodology for immune cytopenia treatments and treatment of autoimmune tissue diseases.

### BACKGROUND OF THE INVENTION

It is well established in the field that IVIg and anti-D are derived from human source material. This obviously presents health risk and economic issues. The danger for transmission of infectious blood diseases clearly exists, which is exacerbated by significant side effects attributable to use. In respect of the economics, extraction and other processing of the compounds is involved and given that large quantities (grams per kilogram of body weight) are necessary for treatment, costs escalate commensurately.

Rampersad et al, in Transfusion, Volume 45, March 2005, investigated the in vitro affects of nitrophenyl compounds as related to specific sulfur redox reactions. The conclusion was drawn that mechanisms which target sulfhydryl groups on mononuclear phagocytes may present therapeutic benefit in the treatment of immune cytopenias.

It has been recently shown that certain chemical compounds containing para-nitrophenyl and sulfur-reactive substituent groups can inhibit FcγR-mediated phagocytosis *in vitro* and may pose promising drug candidates for future treatment of immune cytopenias, Rampersad et al., in Transfusion, 2005; 43:1-9; and Foo et al. in Transfusion, 2007; in press. The mechanism of action of these compounds has been proposed to involve indirect interference of the interaction of FcγR with antibody-coated red blood cells by steric hindrance after binding to thiol groups on the surface of monocyte-macrophages (M_{ϕ}) within close proximity to FcγRs, Woodruff et al., Lancet, 1986; 2:217-8. Immunoglobulins, in contrast, have been shown to inhibit FcγR interaction with antibody-coated cells by directly binding to the FcγR resulting in 'blockade' of this interaction.

What is currently absent from the immunohematology field is a group of compounds and protocol for the use of these for the treatment of autoimmune or alloimmune diseases. The present invention is directed to addressing this need.

### SUMMARY OF THE INVENTION

Accordingly, an object of the present invention is to provide a method for inhibiting phagocytosis of blood cells, comprising providing a nitrophenyl compound and administering said compound to a host having an auto or alloimmune disease for the inhibition of said phagocytosis of blood cells such as red cells, platelets and granulocytes. Examples of the auto or alloimmune disease include, but are not limited to thrombocytopenia, hemolytic anemia, immune cytopenia, rheumatoid arthritis, multiple sclerosis, myasthenia gravis, *inter alia.*

Another object of one embodiment of the present invention is to provide a method for inhibiting phagocytosis of blood cells, comprising providing a thimerosal compound and administering said compound to a host having an auto or alloimmune disease for the inhibition of said phagocytosis of blood cells.

A further object of one embodiment of the present invention is to provide a composition for inhibiting phagocytosis of blood cells, comprising p-nitrophenyl methyl disulfide.

According to another object of one embodiment of the present invention there is provided a composition for inhibiting phagocytosis of blood cells, comprising p-nitrophenylethanol.

According to a still further object of one embodiment of the present invention there is provided a composition for inhibiting phagocytosis of blood cells, comprising thimerosal.

. A still further aspect of one embodiment of the present invention is to provide a composition for inhibiting phagocytosis of blood cells, comprising an immunoglobulin preparation selected from at least one of IVIg or anti-D and a nitrophenyl compound or thimerosal, said preparation and said compound being present in an amount sufficient to effect inhibition of said phagocytosis.

A further object of one embodiment of the present invention is to provide a method for inhibiting tissue destruction due to an autoimmune disease, comprising providing a nitrophenyl compound or thimerosal to a host having an autoimmune disease for the inhibition of said tissue destruction.

These and other features, aspects and advantages of the present invention will become better understood with regard to the following description and accompanying drawings.

### BRIEF DESCRIPTION OF THE DRAWINGS

Figure 1A is a graphical representation of the percentage of inhibition of *in vitro* phagocytosis for a titration of Slide anti-RhD (n=8);

Figure 1B is a graphical representation of the percentage of inhibition of *in vitro* phagocytosis for a titration of WinRho anti-RhD (n=3); .

Figure 1C is a graphical representation of the percentage of inhibition of *in* vitro phagocytosis for a titration of IVIg (n=9);

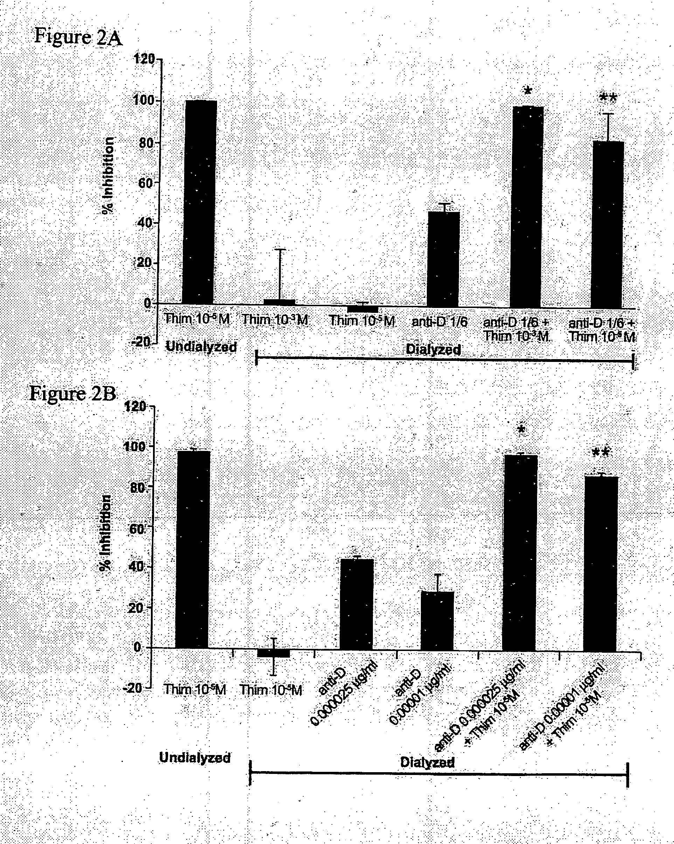

Figure 2A (Slide anti-RhD) is a graphical representation of the mean percentage inhibition of phagocytosis by undialyzed thimerosal at 10⁻⁵M (n=9) and dialyzed thimerosal at 10⁻⁵M (n=9) and 10⁻³M (n=9), anti-RhD at 1/6 dilution (n=9), and anti-RhD at 1/6 dilution that had been mixed with thimerosal at 10⁻⁵M (n=9) and 10⁻³M (n=9) for 24 hours prior to dialysis. Each bar represents 3 independent experiments and standard error of the mean is represented by error bars. The p-values indicate the statistically significant difference between anti-RhD and anti-RhD + thimerosal 10⁻⁵M, and between anti-RhD and anti-RhD + thimerosal 10⁻³M; *p=0.0005; **p=0.0004.

Figure 2B (WinRho anti-RhD) is a graphical representation of the mean percentage inhibition of phagocytosis by dialyzed thimerosal at 10⁻⁵M (n=3), WinRho anti-RhD at 0.000025 mg/mL (n=3), 0.00001 mg/mL (n=3), 0.000025 mg/mL + thimerosal 10⁻⁵ M (n=3) and 0.00001 mg/mL + thimerosal 10⁻⁵ M (n=3); *p=0.003; **p=0.0386.

Figure 3 illustrates FACS analysis of viable, early and late apoptotic monocytic THP-1 cells following treatment with dialyzed slide anti-RhD at 1/6 dilution or anti-RhD (1/6) that had been previously mixed with thimerosal at 10⁻⁵M or 10⁻³M compared to untreated cells. Annexin V-FITC fluorescence is represented on the horizontal axis and PI fluorescence is shown on the vertical axis. The viable, early apoptotic, late apoptotic and necrotic cells are found in the lower left, lower right, upper right and upper left quadrants, respectively. Percentage of cells within each quadrant is indicated;

Figures 4A through 4C is a graphical representation of the data generated from a model of the immune mediated platelet destruction with anti platelet administration over time;

Figure 5 is a graphical representation of treatment as a function of platelet count;

Figure 6 is an illustration of the chemical structures of the compounds used;

Figure 7A is a graphical representation of the mean percent inhibition of phagocytosis by chemicals benzoylmethyl methyl disulfide compared to benzoylmethyl mercaptan;

Figure 7B is a graphical representation of the mean percent inhibition of phagocytosis by chemicals *p*-nitrophenyl methyl disulfide compared to phenyl methyl disulfide and p-nitrobenzyl methyl sulfide;

Figures 8A is a graphical representation of the mean phagocytic index of in vitro Mϕ treated with *p*-nitrophenylethanol (n=6);

Figures 8B is a graphical representation of the mean phagocytic index of in vitro M_{ϕ} treated with p-nitrophenol (n=6);

Figures 8C is a graphical representation of the mean phagocytic index of in vitro M_{ϕ} treated with nitrobenzene (n=6);

Figures 8D is a graphical representation of the mean phagocytic index of in vitro M_{ϕ} treated with 1-phenylethanol (n=6); and

Figure 9 illustrates FACS analysis of viable, early, and late apoptotic cells after treatment with benzoylmethyl methyl disulfide or phenyl methyl disulfide over the concentration range 10⁻⁴ to 10⁻⁹ mol per L.

It will be noted that throughout the appended drawings, like features are identified by like reference numerals.

### DETAILED DESCRIPTION OF THE PREFERRED EMBODIMENT

The human monocytic cell line THP-1 (ATCC 202, Manassas, VA, USA) was maintained in continuous culture in RPMI-1640 (Gibco/Invitrogen, Burlington, Ontario, Canada) containing 10% FBS (Sigma-Aldrich, Oakville, Ontario, Canada) and 0.1% gentamycin (Gibco/Invitrogen) at 37°C and 5% CO₂. THP-1 is a non-adherent leukemia cell line that is phagocytic and contains FcγRs but no cytoplasmic immunoglobulins, Tsuchiya et al., Int. J Cancer, 1980; 26(2):171-6. Normal human peripheral blood was obtained from volunteers. Thimerosal was purchased from BioShop Canada Inc., Burlington, ON, Canada. Human polyclonal anti-D for Slide and Tube Reagent Tests was obtained from Immucor, Houston, Texas, USA. WinRho SDF anti-D was obtained from Cangene. GAMMAGARD S/D Immunoglobulin Intravenous (Human) Therapy (IVIg) (Baxter, Illinois, USA) was obtained from Canadian Blood Services.

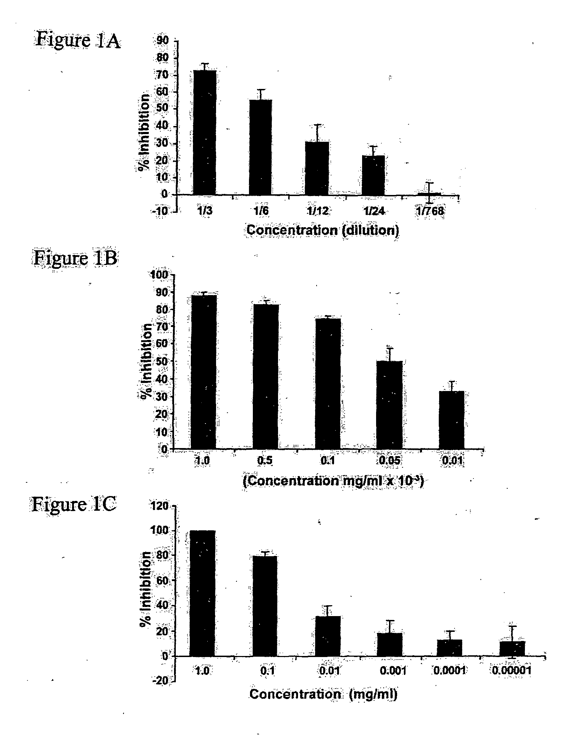

The test concentration of immunoglobulin (anti-D or IVIg) was chosen after titration of each immunoglobulin on its own to inhibit FcγR and phagocytosis of anti-D-coated RBCs using a monocyte monolayer assay (MMA) as previously described in Rampersad et al., *supra* and Foo et al., *supra.* Based on these dose-response inhibitory titration curves, a concentration for each immunoglobulin was chosen so as to have a half-maximum (50%) inhibitory effect. These concentrations were a 1/6 dilution for slide and tube reagent test anti-D, 0.025x10⁻³ mg/ml for WinRho SDF anti-D, and 0.05 mg/ml for IVIg (Figure 1). The two controls used in these experiments were chemical alone and immunoglobulin alone. For each experiment, the controls and a mixture of chemical and immunoglobulin in phosphate buffered saline (PBS) pH 7.4, were placed in separate tubes (Sarstedt) and gently rotated for 24 hours at room temperature. The solutions were then transferred to respective cellulose dialysis tubing membranes with a molecular size cut off of 12,000 daltons (Sigma-Aldrich) that had been cut to length and washed as per the manufacturer's directions by boiling in a solution containing one mmol/L ethylenediaminetetraacetic acid (EDTA) (BioShop) and 2% sodium bicarbonate (Fisher Scientific) in PBS. Dialysis was performed in large beakers of PBS for 5-7 days at 4°C with daily changes of PBS. Following dialysis, the MMA was utilized to determine if free thimerosal had dialysed out of the tubing and was no longer able to inhibit phagocytosis compared to an undialyzed thimerosal control, and the effect of the combination of thimerosal with immunoglobulin was compared to immunoglobulin alone on the ability to inhibit Mϕ phagocytosis.

Preparation of anti-D-sensitized R₂R₂ red blood cells (RBCs) has been previously described in Rampersad et al., *supra* and Foo et al., *supra.* Briefly, RBCs were resuspended in PBS to 5% concentration, mixed with an equal volume of human polyclonal anti-D (Immucor, Texas, Houston, USA) in PBS solution, and then incubated for 1 hour at 37°C and 5% CO₂ After which, the sensitized RBCs were washed four times in PBS and resuspended to 2.5% in PBS. Before the RBC suspension was mixed with an equal volume of culture medium (RPMI-1640 (Gibco/Invitrogen) supplemented with 10% (vol/vol) fetal bovine serum (Sigma-Aldrich) and 20 mM Hepes buffer, pH 7.4 (Gibco/Invitrogen)), an indirect antiglobulin test (IAT) was performed to assess the level of antibody coating of the RBCs and yielded a 4+ reaction. The MMA was performed as previously described in Foo et al., *supra,* without modification.

A phagocytic index was calculated as described in Rampersad et al., *supra;* Foo et al., *supra;* and Branch et al., British Journal of Haematology, 1984; 56:19-29, as the unitless number of antibody-sensitized RBCs phagocytosed per 100 Mϕ. Residual and phagocytosed RBCs were distinguished by relative differences in refracted light under phase-contrast microscopy. Percent inhibition was calculated as previously described² taking the phagocytic control index to be 100. The means and standard error of the mean (SEM) of the results from several independent experiments were determined and analysed statistically. Statistical significance of inhibition between treated and untreated M_{ϕ} were analysed using Student *t*-test and Analysis of Variance (ANOVA), and/or, General Linear Model (GLM) Analysis and Student-Newman-Keuls test. A p value of <0.05 was considered to be significant.

Thimerosal at 10⁻⁵M or 10⁻³M was mixed with slide and tube reagent anti-D used at a 1/6 dilution as illustrated in Figure 2A, or WinRho SDF anti-D, Figure 2B, used at a concentration of 0.025 x 10⁻³ mg/ml to give an approximate 50% inhibitory activity on phagocytosis.

As shown in Figure 2, using the chemical plus immunoglobulin interaction protocol, both anti-D preparations at the concentrations tested maintained their ability to inhibit phagocytosis *in vitro* by approximately 50% after dialysis. However, anti-D at the same concentration that had been mixed with thimerosal at 10⁻⁵M or 10⁻³M was able to inhibit phagocytosis by approximately 83% (p=0.0005) and 100% (p=0.0004), respectively after dialysis for slide anti-D and by approximately 97% (p=0.003) and 89% (p=0.0386) for WinRho SDF anti-D. The statistically significant difference in efficacy between anti-D alone and chemically-treated anti-D was not attributed to effects of free thimerosal as evident in Figure 2. Although thimerosal used alone at 10⁻⁵M or 10⁻³M inhibits phagocytosis by 100% (Figure 2), it no longer inhibits phagocytosis after dialysis (Figure 2) indicating the free thimerosal had been removed from the dialysis tubing and hence the sample.

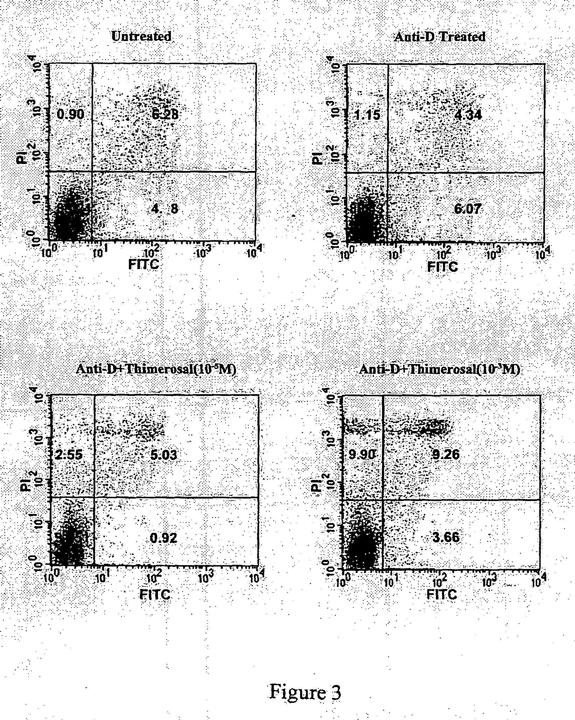

Chemically treating different preparations of anti-D with thimerosal was found to enhance the ability of anti-D to inhibit *in vitro* phagocytosis by up to 100% (Figure 2). Therefore, FACS analysis and Annexin V-FITC Apoptosis Detection Kit (R&D Systems) were used to evaluate the effect of anti-D and thimerosal-treated anti-D on human monocytic THP-1 cell viability and apoptosis. Given the viable cell counts of untreated and treated cells, it was determined that treatment with anti-D at a 1/6 dilution or anti-D that had been mixed with 10-⁵M thimerosal did not significantly alter cell viability or apoptosis (Figure 3). It was noted that when anti-D was mixed with thimerosal at 1O⁻³M and used to treat THP-1 cells, there was a 12.5% decrease in viable cell count compared to untreated THP-1 cells (Figure 3).

### Structure - functional analysis - in vitro chemical inhibition of FCγ-receptor - mediated phagocytosis

### Preparation of p-nitrobenzyl methyl sulfide

Sodium metal (0.53 g, 23 mmol) was dissolved in methanol (100 mL) and p-nitrobenzyl mercaptan (3.94 g, 23 mmol) was added. The deep red solution was cooled with an ice-water bath. Methyl iodide (3.98 g, 28 mmol) in methanol (10 mL) was added dropwise over 3 minutes. The purple reaction mixture was stirred at ambient temperature for 24 hours.

Water (110 mL) was added and the resultant mixture was extracted with chloroform (three 100-mL aliquots). The combined organic layers were dried (MgSO₄) and filtered and the solvent was evaporated. Crude product was chromatographed on silica gel (400 g) employing 2:1 petroleum ether:chloroform (100 mL fractions). Fractions 36 to 57 were combined and concentrated, affording impure *p*-nitrobenzyl methyl sulfide (1.45 g). Impure chromatographed product was rechromatographed on a four-bundle system, Kabir et al., J. Sulfur Chem, 2005; 26:7-11, employing petroleum ether. Fractions 155 to 209 were combined and concentrated, affording clean *p-*nitrobenzyl methyl sulfide (0.46 g, 2.5 mmol, 11%). *p*-Nitrobenzyl methyl sulfide had infra-red 1519 and 1347 per cm. ¹H nuclear magnetic resonance (270 MHz): δ 1.98 (s, 3H), 3.72 (s, 2H), 7.45 (d, 2H), 8.17 (d, 2H). Gas chromatography-mass spectrometry (Rₜ = 8.5 min): 183 (100%, M⁺), 136 (98%), 106 (38%), 89 (53%), 78 (60%).

### Preparation of benzoylmethyl mercaptan

Benzoylmethyl methyl disulfide or phenacyl methyl disulfide, Griffiths et al., Aust J Chem, 2005; 53:1-5, (0.21 g, 1.06 mmol) was added to dry methylene chloride (4 mL). Thiophenol (0.25 g, 2.27 mmol) and pyridine (0.1 mL) were added to the reaction mixture. The resultant solution was stirred at ambient temperature for 2 hours. The solvent was evaporated and the mixture was chromatographed on silica gel (10 g) employing 3:2 petroleum ether:chloroform (5 mL fractions) for elution. Fractions 7 to 15 were combined and concentrated.

The concentrate was dissolved in chloroform (50 mL) and the resultant solution was extracted with 2.5 percent (w/v) sodium hydroxide (four 25-mL aliquots). The combined aqueous layers were set aside, the organic layer was dried (MgSO₄) and filtered, and the solvent was evaporated, affording unchanged benzoylmethyl methyl disulfide (0.077 g, 37%).

The combined aqueous layers were acidified with concentrated hydrochloric acid (15 mL) and the resultant was mixture extracted with chloroform (four 50-mL aliquots). The combined organic layers were dried (MgSO₄) and filtered and the solvent was evaporated yielding benzoylmethyl mercaptan (0.091 g, 0.59 mmol, 56%). Benzoylmethyl mercaptan had infrared 2580, 1680 per cm. ¹H nuclear magnetic resonance (270 MHz): δ 2.14 (t, 1H, J = 8.1 Hz), 3.96 (d, 2H, J = 8.1 Hz), 7.48 (t, 2H), 7.60 (t,1H), 7.96 (d, 2H). ¹³C nuclear magnetic resonance: δ 31.15, 128.51, 128.82, 133.63, 135.01, 194.74. Gas chromatography-mass spectrometry (Rₜ = 6.25 min): 105 (100%), 77 (62%).

### RBC sensitization

As previously described with minor modification, Rampersad et al., Transfusion, 2005; 45:384:93, an aliquot of R₂R₂ RBCs was removed from storage in Alsever's solution, Walker et al., American Association of Blood Banks, 11th ed., Bethesda, 1993, at 4°C and washed three times in PBS without calcium or magnesium (Gibco/lnvitrogen) at 2000 r.p.m. for 7 minutes (Sorvall RT 6000D centrifuge, Mandel Scientific Company Inc., Guelph, Ontario, Canada). The RBCs were resuspended in PBS to 5 percent concentration, mixed with an equal volume of human anti-D in PBS solution, and then incubated for 1 hour at 37°C and 5 percent CO₂ (Sanyo CO₂ incubator), after which the sensitized RBCs were washed four times in PBS at 2000 r.p.m. for 7 minutes and resuspended to 2.5 percent in PBS. Before the RBC suspension was mixed with an equal volume of culture medium, an indirect antiglobulin test was performed to assess the level of antibody coating of the RBCs and yielded a 4+ reaction.

### Monocyte monolayer assay

As previously described with slight modification to improve the quality of the M_{ϕ} monolayer, Rampersad et al, *supra,* whole venous blood was drawn from donors into tubes (Vacutainer, ACD solution A, Becton Dickinson Vacutainer Systems, Franklin Lakes, NJ) and mixed with an equal volume of PBS. In 50-mL tubes (Sarstedt Inc., Montreal, Quebec, Canada), 35 mL of the blood mixture was overlayed onto 15 mL of Ficoll-Paque separation medium (GE Healthcare, Baie d'Urfé, Quebec, Canada), and peripheral blood mononuclear cells (PBMCs) were isolated with density gradient centrifugation at 1800 r.p.m. for 25 min. The PBMCs were washed three times in PBS heated to 37°C, centrifuged at 1200 r.p.m. for 15 minutes and resuspended in culture medium. Viable cell concentration was then adjusted to approximately 2 x 10⁶ cells per mL. One milliliter of this suspension was overlayed onto each 22 x 22-mm coverslip (Fisher Scientific, Waltham, MA) in respective 35 mm petri dishes (Sarstedt Inc.). After 1 hour of incubation at 37°C and 5 percent CO₂, coverslips were washed in PBS that had been warmed to 37°C and placed in new petri dishes with the mononuclear monolayer side facing upward. One milliliter of chemical solution was then overlayed onto each coverslip. For each chemical, the concentrations 10⁻⁴, 10⁻⁵, 10⁻⁶, 10⁻⁷, 10⁻⁸, and 10⁻⁹ mot per L were tested in triplicate. For the positive control, culture medium alone was used in place of drug treatment. Following another incubation period of 1 hour, coverslips were washed gently in 37°C PBS, transferred to new petri dishes with the monolayer side facing upward, and overlayed each with 1 mL of anti-D-sensitized RBC solution. The cells were then incubated for 2 hours at 37°C and 5 percent CO₂, washed gently in 37°C PBS, and air-dried, after which coverslips were first fixed with methanol and then mounted to glass slides with elvanol (20 g of polyvinyl alcohol resin [Sigma-Aldrich] was dissolved in 80 mL PBS at 70°C in a water bath. Afterward, the solution was cooled and mixed thoroughly with 40 mL of glycerin (ICN Biomedicals, Inc., Aurora, OH; final pH was between 6.6 and 7.0). Visual analysis was per-formed by phase contrast microscopy as described previously, Rampersad, *Supra.*

### Fluorescence-activated cell sorting analysis for viable and apoptotic cells

Fluorescence-activated cell sorting (FACS) analysis (flow cytometry) and a TACS annexin V-fluorescein isothiocyanate (FITC) apoptosis detection kit (R&D Systems, Minneapolis, MN) were used to determine if disulfide-containing compounds benzoylmethyl methyl disulfide and phenyl methyl disulfide exert effects on viability or apoptosis of PBMCs. Briefly, PBMCs were isolated from whole blood as previously outlined and treated with culture medium or chemical from 10⁻⁴ to 10⁻⁹ mol per L for 1 hour at 37°C and 5 percent CO₂. They were then washed three times in PBS and collected by centrifugation at 12,000 r.p.m. for 5 to 10 minutes before being incubated in culture medium for 2 hours. After washing the cells once in cold PBS, according to the manufacturer's instructions, cells were then incubated with annexin V-FITC incubation reagent for 15 minutes at room temperature to stain membrane exposed phosphatidylserine, indicating early programmed cell death or apoptosis. Cells were then stained with propidium iodide (PI), specific for nonviable cells, to identify late apoptotic cells. FACS was performed with two-color analysis on a flow cytometer (FACSCalibur E4795, Becton Dickinson, Mississauga, Ontario, Canada) calibrated with fluorescent beads (CaliBRITE, BD Biosciences, San Jose, CA) and computer software for data analyses (Cell Quest, BD Biosciences).

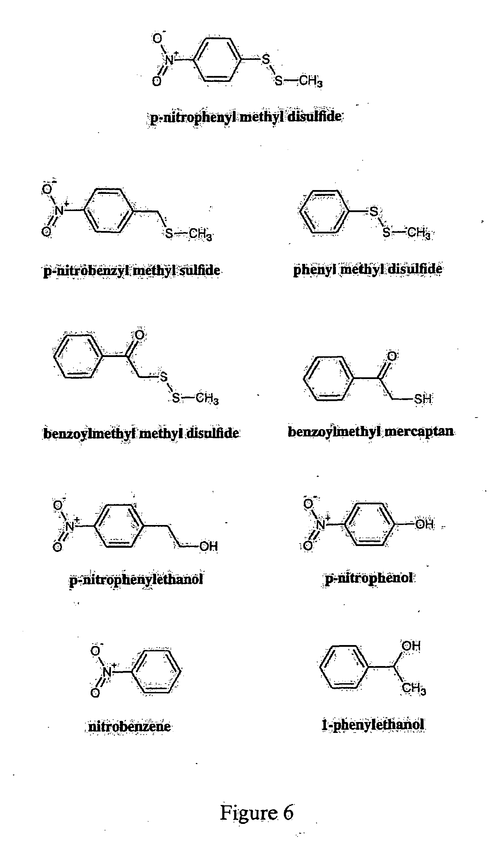

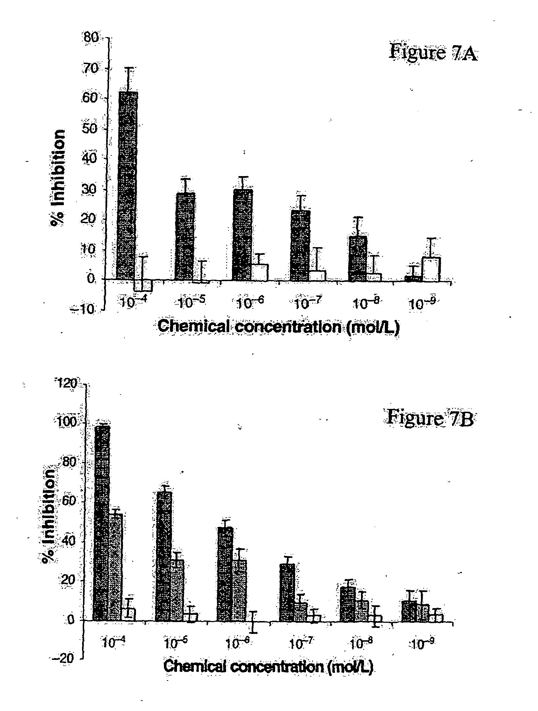

To establish that a disulfide bond is one requirement for a compound to have efficacy for FcγR blockade, the activities of benzoylmethyl methyl disulfide and benzoylmethyl mercaptan were tested and compared as illustrated in Figure 7A. The two compounds are structurally similar where the former contains a reactive disulfide functional group and the latter contains a sulfhydryl moiety, unable to react directly with free sulfhydryl groups on M_{ϕ} (Figure 6). As expected, the disulfide-containing chemical, benzoylmethyl methyl disulfide, inhibited phagocytosis in a dose-dependent manner whereas the sulfhydryl containing benzoylmethyl mercaptan was ineffective (Figure 7A). This indicated that thiol groups are the critical targets of the effective compounds. Interestingly, benzoylmethyl methyl disulfide inhibited phagocytosis by only 62 percent at 10⁻⁴ mol per L (p = 0.004; Figure 7A) and was found to be less effective than the lead compound from the previously published study, p-nitrophenyl methyl disulfide, Rampersad et al., *supra.*

To further confirm the importance of a disulfide moiety, the lead compound selected was *p*-nitrophenyl methyl disulfide. Activity was compared to that of a structurally similar compound synthesized to lack the disulfide moiety, *p*-nitrobenzyl methyl sulfide (Figure 7B). *p-*Nitrobenzyl methyl sulfide is closely related to *p-*nitrophenyl methyl disulfide, the key difference being that replacement of an S with a CH₂ deprives the former of a reactive disulfide bond (Figure 6). From test concentrations of 10⁻⁴ mol per L down to 10⁻⁹ mol per L, *p*-nitrophenyl methyl disulfide, as previously shown, Rampersad et al., *supra,* inhibited macrophage phagocytosis in vitro in a dose-dependent manner (Figure 7B). Results with *p*-nitrophenyl methyl disulfide showed inhibition of phagocytosis by 98.6 percent at 10⁻⁴ mol per L (p = 0.00006) and by 29 percent at 10⁻⁷ mol per L (p = 0.01). In contrast, *p*-nitrobenzyl methyl sulfide did not inhibit phagocytosis over the same test concentration range (Figure 7B).

To establish that the phenyl group itself induces efficacy for FcγR blockade that is further enhanced by the nitro group, we have tested phenyl methyl disulfide (Figure 6), which retains the aromatic ring but lacks the nitro group. The efficacy of *p-*nitrophenyl methyl disulfide was evaluated in comparison to phenyl methyl disulfide (Figure 7B). Results with phenyl methyl disulfide showed inhibition of phagocytosis in a dose-dependent manner (Figure 7B) establishing significant roles for both the phenyl and the nitro group in our lead compound: *p*-nitrophenyl methyl disulfide. Similarly to benzoylmethyl methyl disulfide, phenyl methyl disulfide was not as effective in vitro as *p*-nitrophenyl methyl disulfide. At 10⁻⁴ mol per L, phenyl methyl disulfide inhibited 54 percent of phagocytosis (p = 0.001).

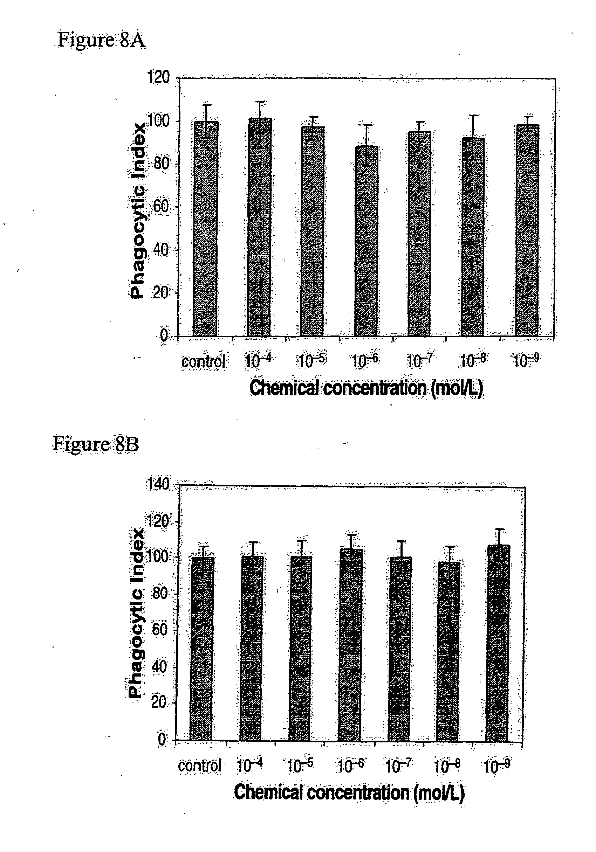

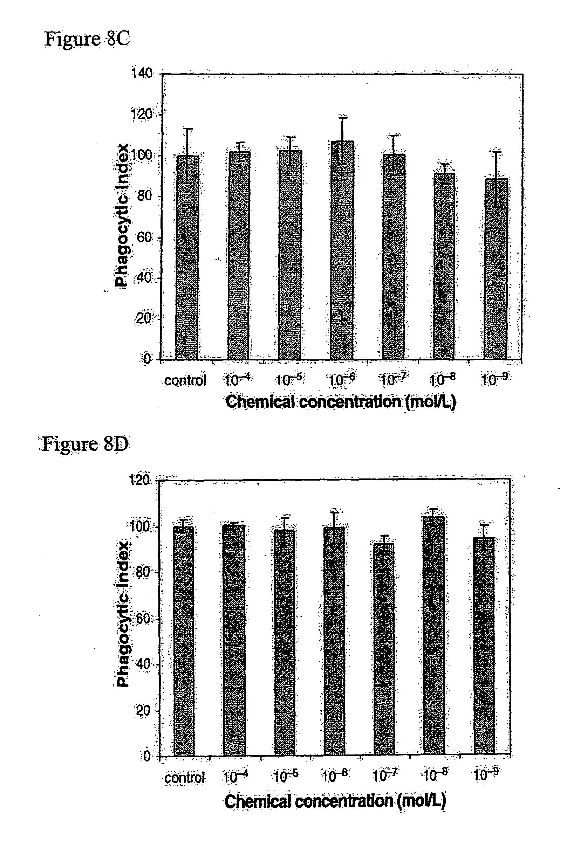

To examine the importance of the reactive *p*-nitrophenyl group and to further elucidate other potentially reactive groups as involved in the chemical inhibition of FcγR-mediated phagocytosis, compounds completely lacking sulfur but containing various combinations of the functional groups were tested, *p*-nitrophenyl and/or hydroxyl moieties (Figure 8). The compounds tested were nitrobenzene, *p*-nitrophenol, *p*-nitrophenylethanol, and 1-phenylethanol (Figure 6). None of these compounds were able to inhibit phagocytosis over a wide concentration range (Figure 8).

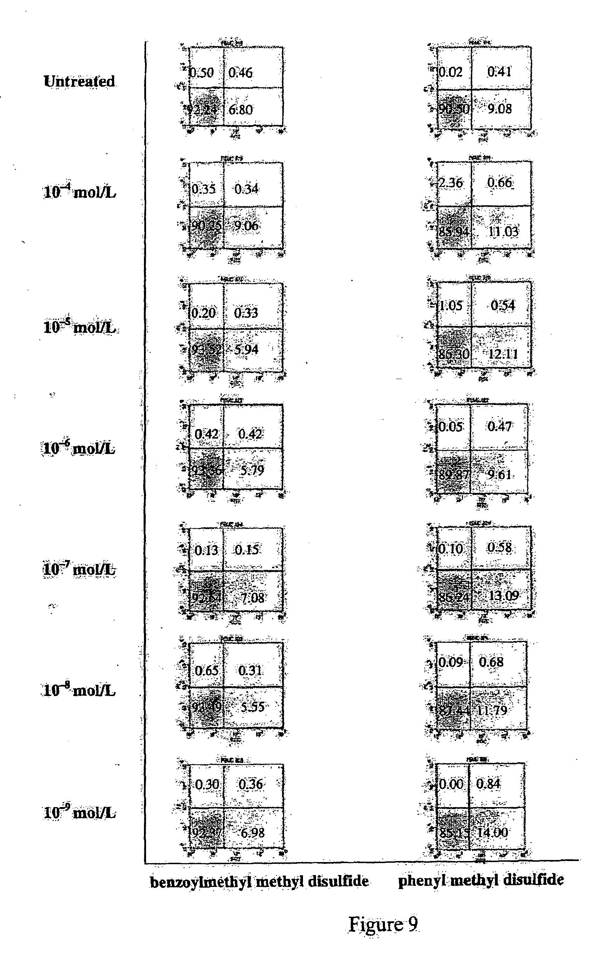

By use of an annexin V-FITC apoptosis detection kit (R&D Systems) and FACS analysis, it was determined that disulfide compounds, benzoylmethyl methyl disulfide and phenyl methyl disulfide, do not significantly affect PBMC viability or apoptosis. Even at concentrations as high as 10⁻⁴ mol per L, benzoylmethyl methyl disulfide-treated PBMCs had viable cell counts comparable to control untreated PBMCs as indicated in the lower left quadrants (Figure. 9). This was the case for PBMCs treated with benzoylmethyl methyl disulfide over a wide concentration range (Figure 9). The viable cell counts for PBMCs treated with phenyl methyl disulfide from concentrations of 10⁻⁴ down to 10⁻⁹ mol per L approximated those for untreated PBMCs (Figure 9). Although the viable cell counts appeared to be unaffected by chemical treatment, it was observed that cells treated with phenyl methyl disulfide at 10⁻⁴ and 10⁻⁵ mol per L had necrotic cell counts of 2.36 and 1.05 percent, respectively, compared to untreated PBMC necrotic cell counts of 0.02 percent as indicated in the top left quadrants of Figure 9.

It has been demonstrated herein that the presence of a disulfide bond is important. As disulfide groups react with free sulthydryl groups, it is believed that any compound that reacts with free sulfhydryl groups has the potential to inhibit FcyR-mediated phagocytosis. A *p*-nitrophenyl group provides enhancement to the efficacy of disulfide-containing compounds.

Mercaptan conjugate bases react with disulfides as shown in Scheme 1.

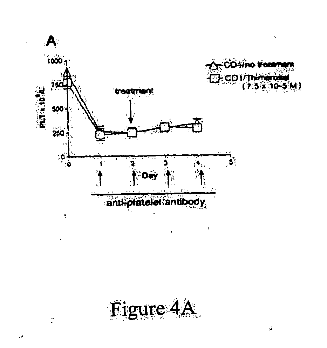

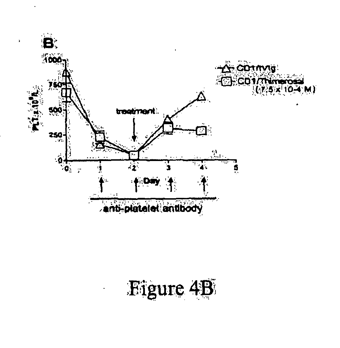

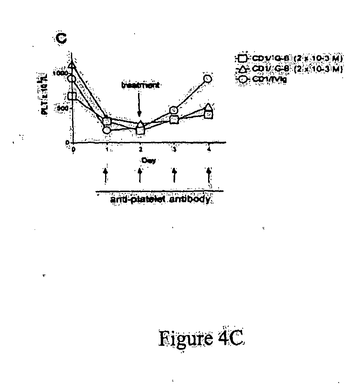

Referring now to Figures 4A through 4C, shown are the results using a model of immune-mediated platelet destruction wherein the anti-platelet antibody is administered on day 0 and daily thereafter. The platelet count fell abruptly after 24 hours (day 1) indicating platelets were destroyed after the antibody was recognized and bound to the platelets. A single dose of IVIg (2 g/kg) at day 2 can reverse the platelet destruction despite the continued administration of anti-platelet antibody (Figures 4B, 4C). In Figures 4B and 4C at day 2, instead of IVIg, two mice were administered a single dose of thimerosal or a nitrophenyl compound p-nitrophenyl methyl disulfide (G-B) (Figure 4C). As is evident, as with IVIg, the platelet count began to rise despite the continued administration of anti-platelet antibody. Although the initial rise in platelet count was almost identical to that seen after IVIg administration, after 48 hours, the platelet count did not increase as with IVIg, but leveled off; however, continued to result in a higher platelet count than the lowest platelet count induced by anti-platelet antibody despite repeated doses of the anti-platelet antibody (Figure 4B) or continued to show a rise in platelet count (Figure 4C).

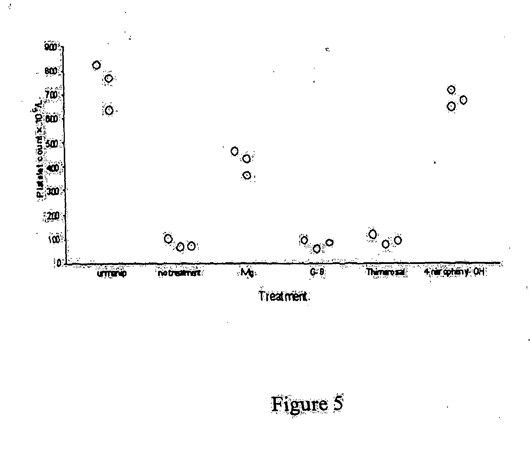

Referring to Figure 5, unlike the model in Figure 4A -4C, the treatment was administered prior to injecting the mice with the anti-platelet antibody. As shown in Figure 5, when three mice in each group were tested before treatment and for each of the treatments, when there was no treatment, the platelet count fell dramatically 24 hours after administration of the anti-platelet antibody. When IVIg was administered prior to giving the anti-platelet antibody, the platelet count fell only to about 50% of initial levels. When G-B or thimerosal were given prior to the anti-platelet antibody, there was little effect on the subsequent loss of platelets follow anti-platelet antibody.

It can be seen that the compound p-nitrophenylethanol (4-nitrophenyl-OH) administered prior to the anti-platelet antibody results in zero platelet loss. In fact, this compound works better than IVIg (2g/kg). The figure is representative of three such experiments having three mice in each group.

It will be understood that numerous modifications thereto will appear to those skilled in the art. Accordingly, the above description and accompanying drawings should be taken as illustrative of the invention and not in a limiting sense. It will further be understood that it is intended to cover any variations, uses, or adaptations of the invention following, in general, the principles of the invention and including such departures from the present disclosure as come within known or customary practice within the art to which the invention pertains and as may be applied to the essential features herein before set forth, and as follows in the scope of the appended claims.

The embodiments of the invention described above are intended to be exemplary only. The scope of the invention is therefore intended to be limited solely by the scope of the appended claims.

## Claims

1. A method for inhibiting phagocytosis of blood cells, **characterized in that** the method comprises:
providing a nitrophenyl compound; and
administering said compound to a host having an auto or alloimmune disease for the inhibition of said phagocytosis of blood cells.

2. The method as set forth in claim 1, **characterized in that** said nitrophenyl compound is an alcohol nitrophenyl compound.

3. The method as set forth in claim 1, **characterized in that** said nitrophenyl compound is p-nitrophenyl methyl disulfide.

4. The method as set forth in claim 1, **characterized in that** said nitrophenyl compound is p-nitrophenylethanol.

5. A method for inhibiting phagocytosis of blood cells, **characterized in that** said method comprises:
providing a thimerosal compound; and
administering said compound to a host having an auto or alloimmune disease for the inhibition of said phagocytosis of blood cells.

6. The method as set forth in claim 1, **characterized in that** said auto or alloimmune disease is an immune thrombocytopenia.

7. The method as set forth in claim 1, **characterized in that** said auto or alloimmune disease is hemolytic anemia.

8. The method as set forth in claim 1, **characterized in that** said auto or alloimmune disease is an immune cytopenia.

9. A composition for inhibiting phagocytosis of blood cells, **characterized in that** said composition comprises:
p-nitrophenyl methyl disulfide.

10. A composition for inhibiting phagocytosis of blood cells, **characterized in that** said composition comprises:
*p*-nitrophenylethanol.

11. A composition for inhibiting phagocytosis of blood cells, **characterized in that** said composition comprises:
thimerosal.

12. A composition for inhibiting phagocytosis of blood cells, **characterized in that** said composition comprises:
an immunoglobulin preparation selected from at least one of IVIg and anti-D; and
a nitrophenyl compound, said preparation and said compound being present in an amount sufficient to effect inhibition of said phagocytosis.

13. The composition as set forth in claim 12, **characterized in that** said nitrophenyl compound comprises *p*-nitrophenyl methyl disulfide.

14. The composition as set forth in claim 12, **characterized in that** said nitrophenyl compound comprises *p*-nitrophenylethanol.

15. The composition as set for thin claim 12, **characterized in that** said composition comprises p-nitrophenyl methyl disulfide and IVIg or anti-D.

16. The composition as set forth in claim 12, **characterized in that** said composition comprises, *p*-nitrophenylethanol and IVIg or anti-D.

17. A composition for inhibiting phagocytosis of blood cells, **characterized in that** said composition comprises:
an immunoglobulin preparation selected from at least one of IVIg and anti-D; and
thimerosal, said preparation and said thimerosal being present in an amount sufficient to effect inhibition of said phagocytosis.

18. A method for inhibiting tissue destruction due to an autoimmune disease, **characterized in that** said method comprises: providing a nitrophenyl compound or thimerosal to a host having an autoimmune disease for the inhibition of said tissue destruction.

19. The method as set forth in claim 18, **characterized in that** said autoimmune disease is rheumatoid arthritis.

20. The method as set forth in claim 18, **characterized in that** said autoimmune disease is multiple sclerosis.

21. The method as set forth in claim 18, **characterized in that** said autoimmune disease is myasthenia gravis.
